# FASCICULE DE BREVET EUROPEEN

(11) **EP 1 568 700 B1**
(45) Date de publication et mention de la délivrance du brevet: **08.08.2007**
(21) Numéro de dépôt: 05290250.9
(22) Date de dépôt: 04.02.2005
(51) Int. Cl.: C07F 7/18, A61K 8/58, A61L 17/04

(54) **Dérivés de s-triazine possédant au moins 2 groupements para-aminobenzalmalonates silaniques; compositions cosmétiques photoprotectrices contenant ces dérivés; utilisations desdits dérivés de s-triazine**
Zumindest zwei p-Aminobenzalmalonatsilangruppen enthaltende s-Triazinderivate, und diese Derivate-enthaltende Sonnenschutzzusammensetzungen, sowie Verwendung dieser s-Triazinderivate
S-triazine derivatives containing at least two silated p-aminobenzalmalonate groups, sunscreen compositions containing these derivatives as well as the use of the derivatives

(30) Priorité: 24.02.2004 FR 0450336
(43) Date de publication de la demande: 31.08.2005
(73) Titulaire: L'ORÉAL, 75008 Paris (FR)
(72) Inventeur: Richard, Hervé, 93420 Villepinte (FR)
(74) Mandataire: Miszputen, Laurent

(56) Documents cités:
- EP-A- 0 841 341

## Description

L'invention concerne de nouveaux dérivés de s-triazine possédant au moins 2 groupements para-amino benzalmalonates particuliers et leurs utilisations en cosmétique.

L'invention concerne également des compositions photoprotectrices, comprenant des dérivés de s-triazine possédant au moins 2 groupements para-amino benzalmalonate greffés à titre de filtres solaires actifs dans le domaine des rayonnements UV.

Dérivés de triazine sont décrits dans le demande de brevet EP 841341.

On sait que les radiations de longueurs d'onde comprises entre 280 nm et 400 nm permettent le brunissement de l'épiderme humain, et que les radiations de longueurs d'onde comprises entre 280 et 320 nm connues sous la dénomination de radiations UV-B, provoquent des érythèmes et des brûlures cutanées qui peuvent nuire au développement du bronzage naturel.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible et/ou continuellement exposée au rayonnement solaire. Les rayons UV-A entraînent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photoallergiques. Ainsi, pour des raisons esthétiques et cosmétiques telles que la conservation de l'élasticité naturelle de la peau, de plus en plus de personnes désirent contrôler l'effet des rayons UV-A sur leur peau. On entend par facteur de protection solaire le rapport du temps d'irradiation nécessaire pour atteindre le seuil érythématogène en présence du filtre testé au temps d'irradiation nécessaire pour atteindre ce même seuil en l'absence de filtre.

Il est donc souhaitable de disposer de composés susceptibles d'absorber les rayons UV-A.

Outre leur pouvoir filtrant du rayonnement UV-A, les composés photoprotecteurs recherchés doivent également présenter de bonnes propriétés cosmétiques, une bonne solubilité dans les solvants usuels et notamment dans les corps gras tels que les huiles et les graisses, ainsi qu'une bonne résistance à l'eau et à la transpiration (rémanence) et une photostabilité satisfaisante.

Parmi tous les composés qui ont été préconisés à cet effet, on peut citer notamment les dérivés de s-triazine portant des substituants benzalmalonates décrits dans la demande EP 0 507 691 de la Demanderesse. Ces composés possèdent cependant une liposolubilité et stabilité photochimique qui ne sont pas encore pleinement satisfaisantes.

La demanderesse a découvert de manière surprenante une nouvelle famille de dérivés de s-triazine portant au moins 2 groupements para-aminobenzalmalonates silaniques ayant de bonnes propriétés absorbantes dans la gamme des rayons UV-A longs et présentant une solubilité dans les corps gras, une photostabilité, ainsi que des qualités cosmétiques nettement améliorées par rapport aux dérivés de s-triazine greffés par des benzalmalonates de l'art antérieur évoqués précédemment.

L'invention concerne une nouvelle famille de dérivés de s-triazine portant au moins 2 groupements para-aminobenzalmalonates particuliers de formule (I) que l'on définira plus loin en détail.

L'invention concerne également une composition cosmétique ou dermatologique, destinée à la photoprotection des matières kératiniques, contenant dans un milieu cosmétiquement acceptable au moins un composé de formule (I).

D'autres objets apparaîtront à la lumière de la description.

Les composés conformes à la présente invention répondent à la formule générale (I) suivante : 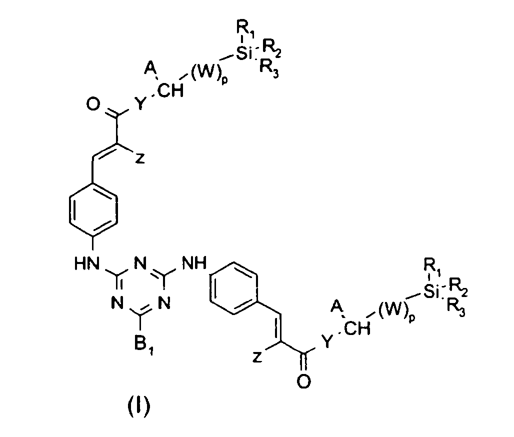 dans laquelle :
- les radicaux R₁, R₂ et R₃ identiques ou différents, représentent un radical alkyle en C₁-C₁₀, linéaire ou ramifié, saturé ou insaturé, pouvant comporter un ou plusieurs atomes d'halogène (par exemple Cl, Br, F) ; un radical phényle,
- p est égal à 0 ou 1,
- A désigne hydrogène ; un radical alkyle en C₁-C₈, linéaire ou ramifié, saturé ou insaturé; un radical phényle ; le groupe Si(CH₃)₃, sous réserve que lorsque A est Si(CH₃)₃, alors p = 0 et R₁, R₂ et R₃ sont méthyle,
- W est un radical alkylène en C₁-C₈, linéaire ou ramifié, saturé ou insaturé, éventuellement substitué par un radical hydroxyle,
- Z désigne -(C=O)OR₄ , -(C=O)R₅, -(C=O)NR₆R₇ , -SO₂R₈, -CN ou
- (C=O)YCHA(W)ₚSiR₁R₂R₃,
- le radical R₄ désigne hydrogène ; un radical alkyle en C₁-C₂₀, linéaire ou ramifié, saturé ou insaturé,
- le radical R₅ désigne un radical alkyle en C₁-C₂₀, linéaire ou ramifié, éventuellement cyclique ; un aryle en C₆-C₁₂,
- les radicaux R₆, R₇, identiques ou différents représentent hydrogène ; un alkyle en C₁-C₂₀, linéaire ou ramifié,
- Y désigne -O- ou -NR₇-,
- le radical R₈ désigne un radical alkyle en C₁-C₂₀, linéaire ou ramifié ; un aryle en C₆-C₁₂.
- B₁ désigne un groupe chromophore choisi parmi ceux du type para amino benzalmalonate, amino benzimidazole, benzimidazole, amino benzoate, amino salicylate, anthranilate, amino benzylidène camphre, amino benzotriazole, amino benzoxazole, para aminophényl benzoxazole ;
- B₁ peut également désigner un groupe aminoalkyle en C₁-C₂₀, linéaire ou ramifié ; un groupe aryle en C₆-C₂₀ substitué ou non par des radicaux alkyles, hydroxy et alkoxy.

Bien que dans la formule (I) ci-dessus, seuls soient représentés les isomères dans lesquels le substituant Z est en position cis par rapport au substituant au noyau aromatique, cette formule doit être comprise comme englobant également l'isomère trans correspondant.

Dans la formule (I) ci-dessus, les radicaux alkyles peuvent être linéaires ou ramifiés, saturés ou insaturés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter-octyle. Le radical alkyle particulièrement préféré est le radical méthyle.

Dans la formule (I) ci-dessus, les radicaux alcoxy sont linéaires ou ramifiés, saturés ou insaturés et choisis de préférence parmi les radicaux méthoxy, éthoxy, n-propyloxy, n-butyloxy.

Dans la formule (I) ci-dessus, les radicaux aryle sont de préférence phényle.

Selon une forme particulière de l'invention , les composés de formule (I) peuvent comporter 3 groupements para-aminobenzalmalonates silaniques à savoir que B₁ désigne un groupe de formule (II) suivante : 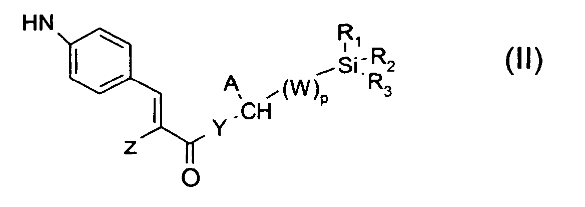 dans laquelle Z, A, p, W, R₁, R₂ et R₃ ont les mêmes significations indiquées précédemment.

Les composés de formule (I) présentent de préférence au moins l'une, et encore plus préférentiellement l'ensemble, des caractéristiques suivantes :
- Z = -(C=O)OR₅ , -CN ou (C=O)YCHA(W)ₚSiR₂R₃R₄,
- R₅ est méthyle ou éthyle,
- A est H,
- R₂ à R₄ désignent alkyle en C₁-C₄ et plus préférentiellement méthyle,
- n est 0,
- p est 0 ou 1,
- W est un radical alkylène en C₁-C₂.

Parmi les composés de formule (I) plus particulièrement préférés, on citera ceux choisis parmi les composés de formules (1) à (6) suivantes :

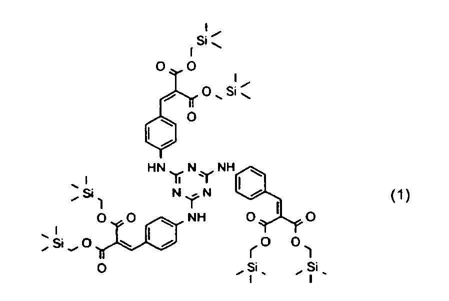

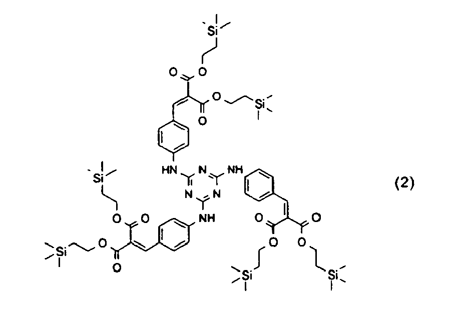

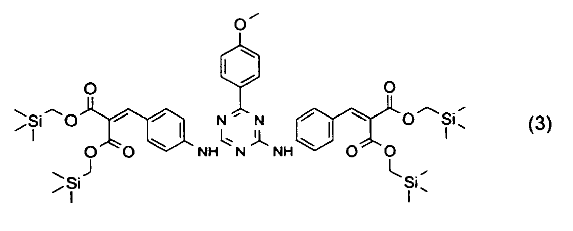

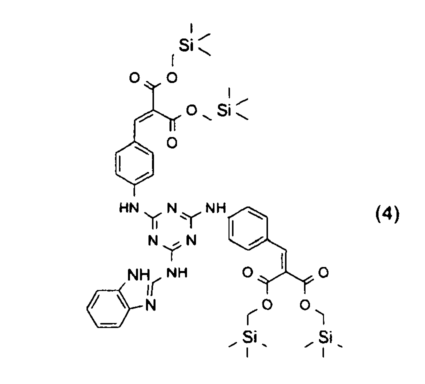

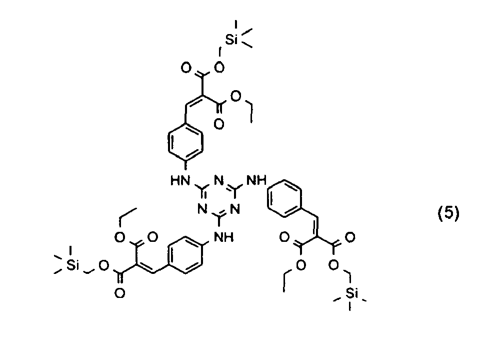

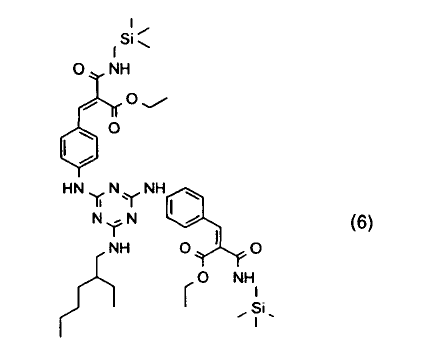

Les composés de formule (I) peuvent être obtenus selon le schéma (a) suivant 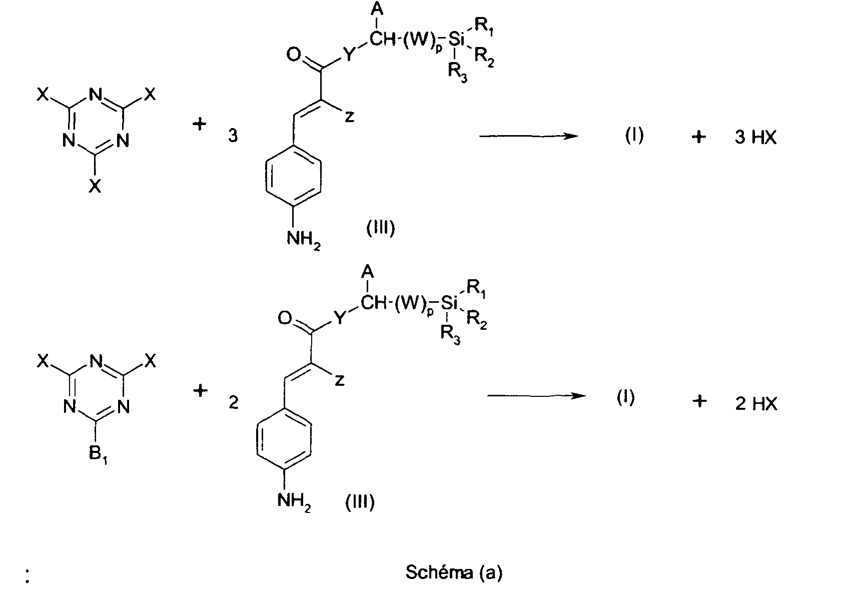 dans lequel R₁, R₂, R₃, A, B₁, Y, W, Z et p ont la définition de la formule (I) ci-dessus et X représente un halogène, en particulier le chlore ou le brome.

Les réactions ci-dessus peuvent être effectuées éventuellement en présence d'un solvant (par exemple : toluène, xylène ou acétone/eau), à une température comprise entre 0°C et 250°C, plus particulièrement entre 5°C et 150°C.

Les composés de formule (III) peuvent être préparés selon des méthodes connues décrites par exemple dans la demande EP 0 507 691 de la Demanderesse.

Dans le cas ou Y est -O-, les composés de la formule (I) peuvent être également obtenus par transestérification de dérivés de formule (IV) selon le Schéma (b) suivant : 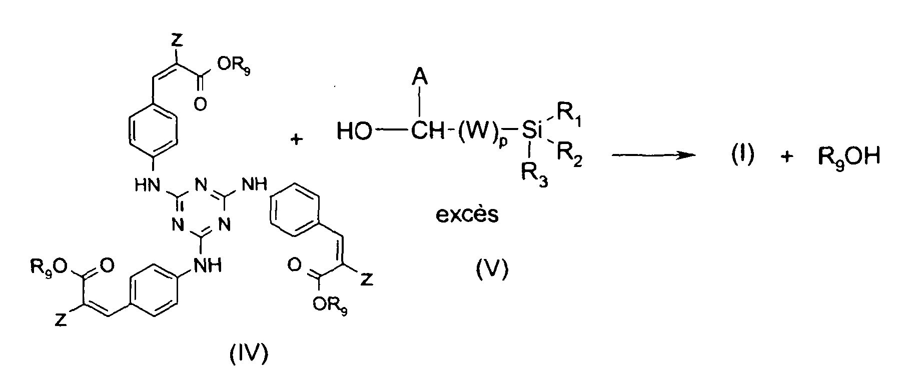 dans lequel R₁, R₂, R₃, A, W, Z et p ont la définition de la formule (I) ci-dessus et R₉ est méthyle ou éthyle.

Les composés de formule (I) sont généralement présents dans la composition de l'invention dans des proportions comprises entre 0,01 % et 20 % en poids, de préférence entre 0,1 % et 10 % en poids, par rapport au poids total de la composition.

Les compositions conformes à l'invention peuvent comporter en plus d'autres filtres UV organiques ou inorganiques complémentaires actifs dans l'UVA et/ou l'UVB hydrosolubles ou liposolubles ou bien insolubles dans les solvants cosmétiques couramment utilisés.

Les filtres organiques complémentaires sont notamment choisis parmi les anthranilates ; les dérivés cinnamiques ; les dérivés de dibenzoylméthane ; les dérivés salicyliques, les dérivés du camphre ; les dérivés de triazine autres que ceux de l'invention tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US 5,237,071, US 5,166,355, GB2303549, DE 197 26 184 et EP893119 ; ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres organiques complémentaires, on peut citer ceux désignés ci-dessous sous leur nom INCI :

### Dérivés de l'acide para-aminobenzoique :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

### Dérivés salicyliques:

Homosalate vendu sous le nom « Eusolex HMS » par Rona/EM Industries,
Ethylhexyl Salicylate vendu sous le nom « NEO HELIOPAN OS » par HAARMANN et REIMER,
Dipropyleneglycol Salicylate vendu sous le nom « DIPSAL » par SCHER,
TEA Salicylate, vendu sous le nom « NEO HELIOPAN TS » par HAARMANN et REIMER,

### Dérivés du dibenzoylméthane :

Butyl Methoxydibenzoylmethane vendu notamment sous le nom commercial « PARSOL 1789 » par HOFFMANN LA ROCHE,
Isopropyl Dibenzoylmethane,

### Dérivés cinnamiques:

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate, DEA Methoxycinnamate, Diisopropyl Methylcinnamate,
Glyceryl Ethylhexanoate Dimethoxycinnamate

### Dérivés de β,β'-diphénylacrylate:

Octocrylene vendu notamment sous le nom commercial « UVINUL N539 » par BASF,
Etocrylene, vendu notamment sous le nom commercial « UVINUL N35 » par BASF,

### Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12 2-(4-diethylamino-2-hydroxybenzoyl)-benzoate de n-hexyle.

### Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,
Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

### Dérivés du phenyl benzimidazole:

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par HAARMANN et REIMER,

### Dérivés de la triazine :

Anisotriazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,

### Dérivés du phenyl benzotriazole :

Drometrizole Trisiloxane vendu sous le nom « Silatrizole » par RHODIA CHIMIE Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

### Dérivés anthraniliques :

Menthyl anthranilate vendu sous le nom commercial commercial « NEO HELIOPAN MA » par HAARMANN et REIMER,

### Dérivés d'imidazolines:

Ethylhexyl Dimethoxybenzylidene Dioxoimidazoline Propionate,

### Dérivés du benzalmalonate :

Polyorganosiloxanes à fonction benzalmalonate tels que le Polysilicone-15 vendu sous la dénomination commerciale « PARSOL SLX » par HOFFMANN LA ROCHE

### Dérivés de 4,4-diarylbutadiène:

-1,1-dicarboxy (2,2'-diméthyl-propyl)-4,4-diphénylbutadiène

### Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom Uvasorb K2A par Sigma 3V ; et leurs mélanges.

Les filtres UV organiques complémentaires préférentiels sont choisis parmi
Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
et leurs mélanges.

Les filtres complémentaires inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les filtres UV complémentaires conformes à l'invention sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions cosmétiques selon l'invention peuvent contenir en outre des agents de bronzage et/ou de brunissage artificiel de la peau (agents autobronzants) tels que la dihydroxyacétone (DHA).

Les compositions conformes à la présente invention peuvent comprendre en outre des adjuvants cosmétiques classiques notamment choisis parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants ou tout autre ingrédient habituellement utilisé dans le domaine cosmétique et/ou dermatologique.
Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Parmi les huiles pouvant rentrer dans la composition de la phase grasse, on peut notamment citer :
- les huiles minérales telles que l'huile de paraffine et l'huile de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques telles que l'huile de purcellin, les esters comme par exemple le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-a-oléfines.

Comme autres huiles utilisables dans les compositions selon l'invention, on peut encore citer les benzoates d'alcools gras en C₁₂-C₁₅ (Finsolv TN de FINETEX), les éthers, les dérivés lipophiles d'acide aminé tels que le N-lauroylsarcosinate d'isopropyl (Eldew SL-205 d'Ajinomoto), les alcools gras tels que l'alcool laurique, cétylique, myristique, stéarique, palmitique, oléique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en C₁₀-C₁₈, les huiles fluorées et perfluorées, la lanoline, la lanoline hydrogénée, la lanoline acétylée et enfin les huiles de silicones, volatiles ou non.

Bien entendu, la phase grasse peut également contenir un ou plusieurs adjuvants cosmétiques lipophiles classiques, comme par exemple des cires, des gélifiants lipophiles, des tensio-actifs, des particules organiques ou minérales, et notamment ceux qui sont déjà utilisés de manière habituelle dans la fabrication et l'obtention des compositions cosmétiques antisolaires.

Comme composés cireux, on peut citer la paraffine, la cire de camauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Les épaississants peuvent être choisis notamment parmi les polymères acryliques réticulés comme les Carbomer, les polymères réticulés acrylates/C₁₀-C₃₀alkylacrylates du type Pemulen ou le polyacrylate-3 vendu sous le nom VISCOPHOBE DB 1000 par Amerchol ; les polyacrylamides tels que l'émulsion polyacrylamide, C₁₃-C₁₄ isoparraffine et laureth-7 vendue sous le nom SEPIGEL 305 par SEPPIC, les homopolymères ou copolymères d'AMPS tel l'HOSTACERIN AMPS vendu par CLARIANT, les gommes de guar et celluloses modifiées ou non telles que la gomme de guar hydroxypropylée, la méthylhydroxyéthylcellulose et l'hydroxypropylméthyl cellulose, la gomme de xanthane, les silices nanométriques de type Aerosil.

Bien entendu, l'homme de l'art veillera à choisir le ou les éventuels composés complémentaires cités ci-dessus et/ou leurs quantités de manière telle que les propriétés avantageuses attachées intrinsèquement aux composés conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, d'une huile, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Lorsque la composition cosmétique selon l'invention est utilisée pour le soin de l'épiderme humain, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, d'huile solaire, de bâtonnet solide, de poudre, de mousse aérosol ou de spray.

Lorsque la composition cosmétique selon l'invention est utilisée pour le soin des cheveux, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des ongles, des lèvres, des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Les compositions selon l'invention peuvent se présenter sous forme de lotions fluides vaporisables conformes à l'invention et être appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Un autre objet de l'invention est l'utilisation d'un composé de formule (I) tel que définie ci-dessus dans une composition cosmétique ou dermatologique comme agent filtrant les radiations UV.

Un autre objet de l'invention est l'utilisation d'un composé de formule (I) tel que définie ci-dessus dans une composition cosmétique comme agent de contrôle de la variation de la couleur de la peau due aux rayonnements UV.

Un autre objet de l'invention est l'utilisation d'un composé de formule (I) tel que définie précédemment comme agent photostabilisant de polymères synthétiques tels que les matières plastiques ou de verres en particulier des verres de lunettes ou des lentilles de contact.

Des exemples concrets, mais nullement limitatifs, illustrant l'invention, vont maintenant être donnés.

### EXEMPLE 1 : Préparation du 2,4,6-tris(4'-amino benzalmalonate de di-méthyltriméthylsilyle)-s-triazine :

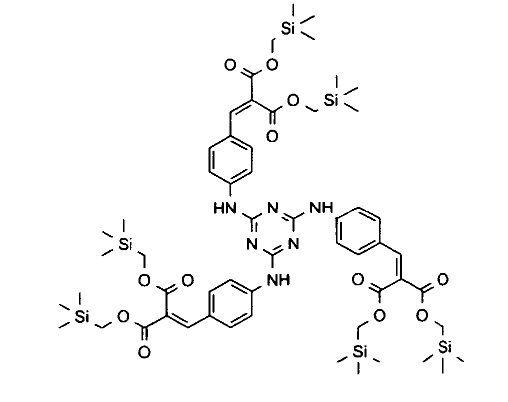

### Première étape : préparation du malonate de di-méthyltriméthvlsilyle :

Dans un réacteur surmonté par un Dean Stark, on porte au reflux pendant 3 heures l'acide malonique (8,3 g, 0,079 mole) et l'alcool méthyltriméthylsilyle (18,35 g, 0,176 mole) dans 50 ml de toluène en présence de 0,1 ml d'acide sulfurique concentré. L'eau formée est éliminée par azéotropie. La phase organique est lavée 3 fois à l'eau et est séchée sur sulfate de sodium. Après filtration et évaporation du solvant sous vide, on obtient 22 g d'une huile incolore. Après distillation sous vide de 0,15 mbar, on recueille les fractions distillant à 74-76°C de malonate de di-méthyltriméthylsilyle (19,6 g, Rendement 90%) sous forme d'une huile incolore et utilisée telle quelle dans l'étape suivante.

### Deuxième étape : préparation du 4-nitrobenzalmalonate de di-méthyltriméthylsilyle :

Dans un ballon équipé d'un Dean Stark surmonté d'un réfrigérant et sous barbotage d'azote, on place le p-nitro benzaldéhyde (4,65 g, 0,03 mole) et le malonate de dinéopentyle (8,5 g, 0,031 mole) dans 15 ml de toluène. On y ajoute le catalyseur préparé par avance, l'acide acétique (0,2 ml) et la pipéridine (0,3 ml) en suspension dans 0,5 ml de toluène. On porte le mélange sous agitation au reflux pendant 5 heures et élimine par l'intermédiaire du Dean Stark l'eau formée. Deux rajouts des mêmes quantités de catalyseur ont été nécessaires. Le toluène est éliminé sous vide, le résidu est repris dans l'éther isopropylique puis lavée 2 fois à l'eau. Après séchage de la phase organique et évaporation du solvant, on obtient une huile rouge marron. Après purification sur colonne de silice (éluant : Heptane/AcOEt 9:1), on recueille 7,1 g (Rendement 57%) de fractions propres de 4-nitrobenzalmalonate de di-méthyltriméthylsilyle sous forme d'un solide jaune pâle et utilisé tel quel dans l'étape suivante.

### Troisième étape : préparation du 4-aminobenzalmalonate de di-méthyltriméthylsilyle :

Sous agitation et barbotage d'azote, on disperse le dérivé de l'étape précédente (3,2 g, 0,0078 mole) dans 5,5 ml d'acide acétique. On y ajoute 8 ml d'eau. Le mélange est chauffé à 50°C. On y ajoute le fer (4,36 g) par portions sans dépasser une température de 55°C (temps d'introduction 1 heure). Ensuite, on ajoute goutte à goutte de l'acide acétique (8 ml) sans dépasser une température de 55°C (temps d'introduction 1 heure). On chauffe 45 minutes de plus à 55°C. On refroidit, ajoute du dichlorométhane et on filtre sur Célite. La phase organique est lavée à l'eau, avec une solution saturée de bicarbonate de sodium, à l'eau puis est séchée sur sulfate de sodium. Après concentration sous pression réduite on obtient une huile orangée qui est purifiée par chromatographie sur colonne de silice (éluant : Heptane/AcOEt 9 :1). On recueille 1,8 g (Rendement 62%) de fractions propres de 4-aminobenzalmalonate de di-méthyltriméthylsilyle sous forme d'un solide jaune et utilisés tel quel dans l'étape suivante.

### Quatrième étape : préparation du dérivé de l'exemple 1 :

Sous barbottage d'azote, le dérivé précédent (1,1 g, 9,66x10⁻⁴ mole) dissout dans 5 ml de toluène est introduit à 0-5°C goutte à goutte à une dispersion de chlorure de cyanuryle (0,18 g, 3,22x10⁻⁴ mole) dans 10ml de toluène. On chauffe ensuite au reflux pendant 5 heures sous dégazage d'azote. Après refroidissement, la phase organique est lavée à l'eau puis par une solution aqueuse de bicarbonate de sodium. On concentre sous pression réduite. Le résidu obtenu est solubilisé dans du 1,2-dichloroéthane. La phase organique est lavée à l'eau, avec une solution saturée de bicarbonate de sodium, à l'eau puis est séchée sur sulfate de sodium. Après concentration sous pression réduite on obtient une huile jaune qui est purifiée par chromatographie sur colonne de silice (éluant : Heptane/AcOEt 85 :15). On recueille 0,75 g (Rendement 64%) de fractions propres du dérivé de l'exemple 1 sous forme d'une gomme jaune pâle :

| | | | |
|---|---|---|---|
| UV (Ethanol) | λₘₐₓ= 355 nm ; | εₘₐₓ= 118 970 ; | E_{1%}= 987. |

### EXEMPLE 2 : Préparation du 2,4,6-tris(4'-amino benzalmalonate de di-éthyltriméthylsilyle)-s-triazine:

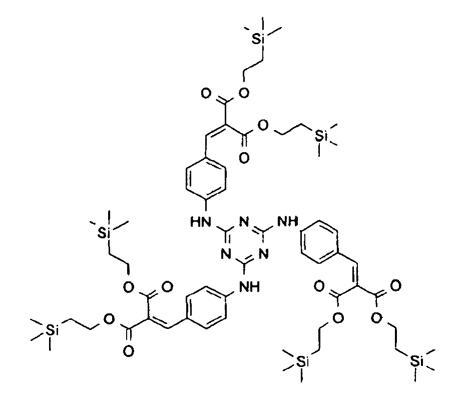

Sous barbottage d'azote, du 2,4,6-tris(4'-amino benzalmalonate de di-éthyle)-s-triazine (1 g, 1,16x10⁻⁴ mole) (Exemple 1 de la demande EP) est dissout dans 5 ml d'alcool méthyltriméthylsilyle. On chauffe ensuite au reflux pendant 25 heures avec 0,2 g d'acide para toluène sulfonique. Après ajout de 30 ml de dichlorométhane, la phase organique est lavée à l'eau puis par une solution aqueuse de bicarbonate de sodium et séchée. On concentre sous pression réduite. Le résidu obtenu est purifiée par chromatographie sur colonne de silice (éluant : Heptane/AcOEt 85 :15). On recueille 0,35 g (Rendement 32%) du dérivé de l'exemple 2 sous forme d'une gomme jaune pâle :

| | | | |
|---|---|---|---|
| UV (Ethanol) | λₘₐₓ = 355 nm ; | εₘₐₓ = 125 250 ; | E_{1%} = 965. |

### EXEMPLE 3 : Préparation du 2,4-bis(4'-amino benzalmalonate de di-méthyltriméthylsilyle)-6-(para-méthoxyphényl)-s-triazine:

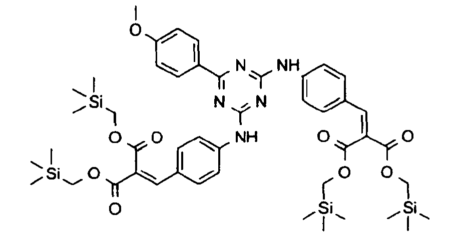

Du 2,4-dichloro-6-(para-méthoxy)-s-triazine (0,67 g, 2,61x10⁻³ mole) est dispersé dans 10 ml de toluène. Une solution dans 10 ml de 4-aminobenzalmalonate de di-méthyltriméthylsilyle (2 g, 5,2x10⁻³ mole) y est ajouté goutte à goutte sous barbotage d'azote. On porte le mélange au reflux pendant 4 heures 30 minutes. Après un ajout de 20 ml de dichlorométhane, la phase organique est lavée à l'eau, avec une solution saturée de bicarbonate de sodium, à l'eau puis est séchée sur sulfate de sodium. La phase organique est concentrée sous vide. Le résidu est soumis à une séparation sur colonne de silice (éluant : Heptane/EtOAc 8 :2). On récupère des fractions propres sous forme d'une huile jaune pâle que l'on précipite dans un mélange Ethanol/Heptane pour obtenir le dérivé de l'exemple 3 (1,42 g, Rendement 58%) sous forme d'une poudre jaune pâle amorphe :

| | | | |
|---|---|---|---|
| UV (Ethanol) | λₘₐₓ = 351 nm ; | εₘₐₓ = 74 540 ; | E_{1%} = 791, |
| | λₘₐₓ = 340 nm ; | εₘₐₓ = 74 450 ; | E_{1%} = 790, |
| | λₘₐₓ = 296 nm ; | εₘₐₓ = 28 280 ; | E_{1%} = 340. |

### EXEMPLE 4 : Préparation du 2,4-bis(4'-amino benzalmalonate de diméthyltriméthylsilyle)-6-(2'-aminobenzimidazole)-s-triazine-

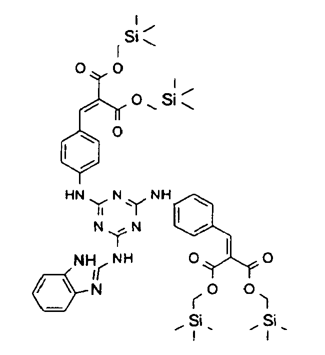

### Première étape :préparation du 2,4-dichloro-6-(2'-aminobenzimidazole)-s-triazine :

Le chlorure de cyanuryle (18 g, 0,097 mole) est solubilisé à 0°C dans 150 ml d'acétone. On y ajoute 50 ml d'eau. Une suspension de 2-aminobenzimidazole (13 g, 0,097 mole) dans 100 ml d'acétone y est ajouté goutte à goutte sous barbotage d'azote à 0°C. On y ajoute une solution de carbonate de sodium (7 g) dans 150 ml d'eau. Le mélange hétérogène est laissé sous agitation à 0-5°C pendant 2 heures. Le mélange réactionnel est filtré. Le solide est lavé au dichlorométhane, à l'eau et avec un peu d'acétone. Après séchage, on récupère 25 g (Rendement 91%) du 2,4-dichloro-6-(2'-aminobenzimidazole)-s-triazine sous forme d'une poudre beige et utilisé tel quel dans l'étape suivante.

### Deuxième étape : préparation du dérivé de l'exemple 4 :

Le dérivé précédent (0,267 g, 9,5x10⁻⁴ mole) est dispersé avec le 4-aminobenzalmalonate de di-méthyltriméthylsilyle méthyltriméthylsilyle (0,72 g, 1,9x10⁻³ mole) dans 10 ml de toluène. On porte le mélange au reflux pendant 4 heures 30 minutes. La phase organique est concentrée sous vide. Après un ajout de 20 ml de dichlorométhane, la phase organique est lavée à l'eau, avec une solution saturée de bicarbonate de sodium, à l'eau puis est séchée sur sulfate de sodium. Après cristallisation dans un mélange dichlorométhane/MeOH, on obtient le dérivé de l'exemple 4 (0,66 g, Rendement 72%) sous forme d'une poudre marron clair amorphe :

| | | | |
|---|---|---|---|
| UV (Ethanol) | λₘₐₓ = 342 nm ;épaulement | εₘₐₓ = 61 910 ; | E_{1%} = 640, |
| | λₘₐₓ = 325 nm ; | εₘₐₓ = 76 420 ; | E_{1%} = 790. |

### EXEMPLE DE FORMULATION DANS UNE EMULSION H/E

| **DENOMINATION CHIMIQUE** | **Composition** |
|---|---|
| **REFERENCE COMMERCIALE- Fournisseur** | **(g %)** |
| MELANGE MONO-STEARATE DE GLYCERYLE / STEARATE DE POLYETHYLENE GLYCOL (100 OE) | 1 |
| **SIMULSOL 165 - Seppic** | |
| ACIDE STEARIQUE | 1.5 |
| **STEARINE TP 1200 PASTILLES - Stearinerie Dubois** | |
| POLY DIMETHYLSILOXANE | 0.5 |
| **200 FLUID 350 CS - Dow Corning** | |
| ALCOOL CETYLIQUE | 0.5 |
| **LANETTE 16 NF - Cognis** | |
| MELANGE CETYLSTEARYL GLUCOSIDE / ALCOOL CETYLSTEARYLIQUE | 2 |
| **MONTANOV 68 - Seppic** | |
| CONSERVATEUR | 1 |
| TRIETHANOLAMINE | 0.45 |
| **TRIETHANOLAMINE - BASF** | |
| TRIGLYCERIDE D'ACIDE CAPRIQUE / CAPRILIQUE 10 | 10 |
| **MYRITOL 317- Cognis** | |
| COMPOSE DE L'EXEMPLE 1 | 5 |
| GLYCERINE | 5 |
| **PRICERINE 9091 - Uniquema** | |
| GOMME DE XANTHANE | 0.1 |
| **KELTROL T - CP kelco** | |
| COPOLYMERE ACIDE ACRYLIQUE / ACRYLATE D'ALKYLE (C10/C30) RETICULE | 0.12 |
| **PEMULEN TR-1 - Noveon** | |
| TRIETHANOLAMINE | qs pH |
| **TRIETHANOLAMINE - BASF** | |
| EAU DEMINERALISEE | qsp 100g |

### PHOTOSTABILITES COMPAREES ENTRE UN COMPOSE DE L'ART ANTERIEUR ET LE COMPOSE SELON L'INVENTION DE L'EXEMPLE 1.

### Produits testés :

2,4,6-tris(4'-amino benzalmalonate de diisobutyle)-s-triazine = art intérieur (exemple 1 du brevet EP507691).
2,4,6-tris(4'-amino benzalmalonate de di-méthyltriméthylsilyle)-s-triazine = Exemple 1 selon l'invention.

Les deux produits ont été mis en solution à 5% en poids dans l'huile Miglyol 812. Environ 10 mg de solution huileuse sont étalés sur 10 cm² à la surface d'un disque creux de verre dépoli ; la quantité est déterminée par pesée.

Les films des solutions huileuses sont irradiés une heure à l'aide d'un si mulateur solaire ORIEL (UVA = 14,2 mW/cm² ; UVB = 0,41 mW/cm²), puis extraits par 10 ml d'éthanol à10% d'isopropanol et passage 5 min aux ultrasons.
Les quantifications des produits sont faites par HPLC des extraits.

Conditions HPLC : colonne : UP5WOD-25QS, 250*4.6 mm, 5 µm, Interchrom; éluant : méthanol (exemple comparatif 1) et 96% de méthanol + 4% d'eau (exemple 1) ; Débit : 1 ml/mn ; volume injecté : 10 µl ; détection : Barrette de diodes ; tr (min ) : 5.2 (exemple comparatif 1) et 13,8 (exemple 1).

Les taux de perte sont déterminés par comparaison des quantités de produit présent dans les échantillons irradiés et dans les témoins non irradiés préparés simultanément et traités de la même façon (moyennes sur 3 échantillons ; S = surface / mg solution) : % perte = 100 * (S0 - Sirr) / S0

### RESULTATS DE PHOTOSTABILITE

| **Composés** | **Essai** | **% de disparition** |
|---|---|---|
| **Composé (art antérieur)** | **1** | **11** |
| **Composé (art antérieur)** | **2** | **10** |
| **Composé (art antérieur)** | **3** | **9** |
| **Exemple 1** | **4** | **0** |
| **Exemple 1** | **5** | **0** |
| **Exemple 1** | **6** | **0** |

Composé de l'art antérieur : La perte dans le Miglyol est comprise entre 9 et 11% après une heure d'exposition au simulateur d'un film de 1mg/cm² de solution à 5%.

Exemple 1 : Aucune perte dans le Miglyol est observée après une heure d'exposition au simulateur d'un film de 1mg/cm² de solution à 5%.

## Revendications

1. Composé répondant à la formule générale (I) suivante : 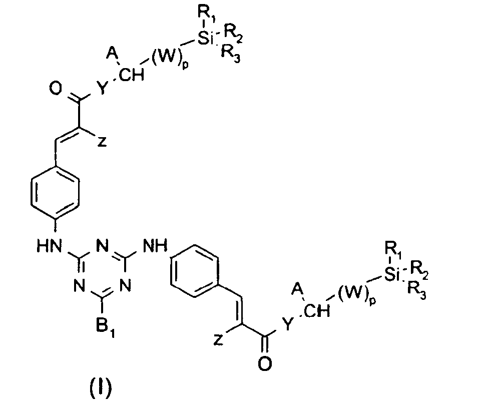 dans laquelle :
- les radicaux R₁, R₂ et R₃ identiques ou différents, représentent un radical alkyle en C₁-C₁₀, linéaire ou ramifié, saturé ou insaturé, pouvant comporter un ou plusieurs atomes d'halogène ; un radical phényle,
- p est égal à 0 ou 1,
- A désigne hydrogène ; un radical alkyle en C₁-C₈, linéaire ou ramifié, saturé ou insaturé; un radical phényle ; le groupe Si(CH₃)₃, sous réserve que lorsque A est Si(CH₃)₃, alors p = 0 et R₁, R₂ et R₃ sont méthyle,
- W est un radical alkylène en C₁-C₈, linéaire ou ramifié, saturé ou insaturé, éventuellement substitué par un radical hydroxyle,
- Z désigne -(C=O)OR₄ , -(C=O)R₅, -(C=O)NR₆R₇ , -SO₂R₈ , -CN ou
- (C=O)YCHA(W)ₚSiR₁R₂R₃ ,
- le radical R₄ désigne hydrogène ; un radical alkyle en C₁-C₂₀, linéaire ou ramifié, saturé ou insaturé,
- le radical R₅ designe un radical alkyle en C₁-C₂₀, linéaire ou ramifié, éventuellement cyclique ; un aryle en C₆-C₁₂,
- les radicaux R₆, R₇, identiques ou différents représentent hydrogène ; un alkyle en C₁-C₂₀, linéaire ou ramifié,
- Y désigne -O- ou -NR₇-,
- le radical R₈ désigne un radical alkyle en C₁-C₂₀, linéaire ou ramifié ; un aryle en C₆-Cₗ₂.
- B₁ désigne un groupe chromophore choisi parmi ceux du type para amino benzalmalonate, amino benzimidazole, benzimidazole, amino benzoate, amino salicylate, anthranilate, amino benzylidène camphre, amino benzotriazole, amino benzoxazole, para aminophényl benzoxazole;
- B₁ peut désigner un groupe aminoalkyle en C₁-C₂₀, linéaire ou ramifié ; un groupe aryle en C₆-C₂₀ substitué ou non par des radicaux alkyles, hydroxy et alkoxy.

2. Composé selon la revendication 1, où B₁ désigne un groupe de formule (II) suivante : 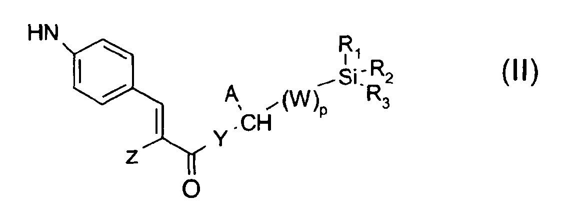 dans laquelle Z, A, p, W, R₁, R₂ et R₃ ont les mêmes significations indiquées dans la revendication 1..

3. Composé selon la revendication 1 ou 2, présentant au moins l'une, et encore plus préférentiellement l'ensemble, des caractéristiques suivantes :
- Z = -(C=O)OR₅ , -CN ou (C=O)YCHA(W)ₚSiR₂R₃R₄,
- R₅ est méthyle ou éthyle,
- A est H,
- R₂ à R₄ désignent alkyle en C₁-C₄ et plus préférentiellement méthyle,
- n est 0,
- p est 0 ou 1,
- W est un radical alkylène en C₁-C₂.

4. Composé selon la revendication 3, choisi parmi les composés suivants :

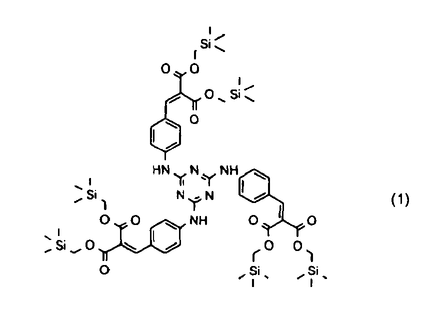

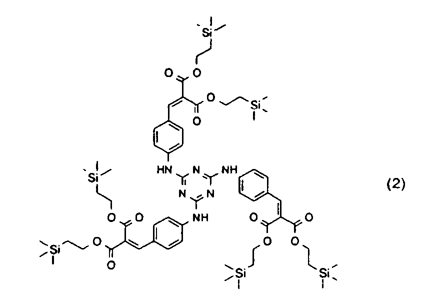

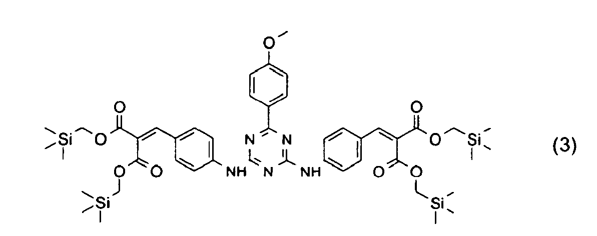

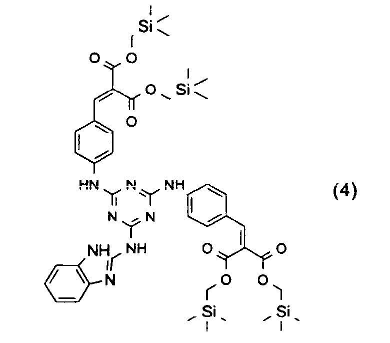

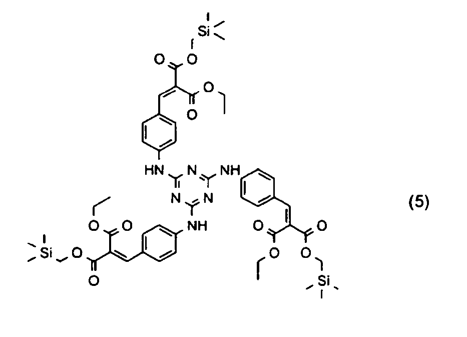

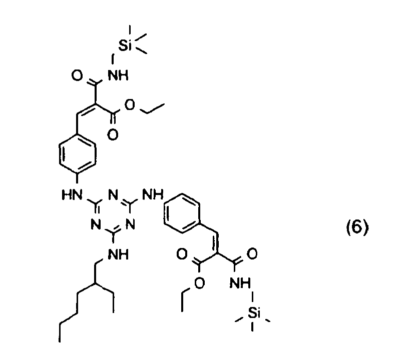

5. Composition cosmétique ou dermatologique, destinée à la photoprotection des matières kératiniques, **caractérisée par le fait qu'**elle contient dans un support cosmétiquement acceptable au moins un composé de formule (I) tel que défini dans l'une quelconque des revendications 1 à 4.

6. Composition selon la revendication 5, **caractérisée par le fait que** le composé de formule (I) est présent dans la composition à une teneur allant de 0,01 à 20 % en poids, par rapport au poids total de la composition, de préférence allant de 0,1 % à 10 % en poids, par rapport au poids total de la composition.

7. Composition selon la revendication 5 ou 6, **caractérisée par le fait que** ledit support cosmétiquement acceptable se présente sous la forme d'une émulsion de type huile-dans-eau ou eau-dans-huile.

8. Composition selon l'une quelconque des revendications 5 à 7, **caractérisée par le fait qu'**elle comprend en outre un ou plusieurs filtres organiques ou inorganiques complémentaires actifs dans l'UV-A et/ou UV-B.

9. Composition selon la revendication 8, **caractérisée par le fait que** lesdits filtres organiques complémentaires sont choisis parmi les anthranilates ; les dérivés cinnamiques; les dérivés de dibenzoylméthane ; les dérivés salicyliques, les dérivés du camphre ; les dérivés de triazine autres que ceux définis dans les revendications précédentes; les dérivés de la benzophénone ; les dérivés de β,β-diphénylacrylate ; les dérivés de benzotriazole ; les dérivés de benzalmalonate autres que ceux définis dans les revendications précédentes ; les dérivés de benzimidazole ; les imidazolines ; les dérivés bis-benzoazolyle ; les dérivés de benzoxazole ;les dérivés de l'acide p-aminobenzoïque (PABA) ; les dérivés de méthylène bis-(hydroxyphényl benzotriazole) ; les dérivés de benzoxazole ; les polymères filtres et silicones filtres; les dimères dérivés d'α-alkylstyrène; les 4,4-diarylbutadiènes et leurs mélanges.

10. Composition selon la revendication 9, **caractérisée par le fait que** lesdits filtres organiques complémentaires sont choisis parmi
Ethylhexyl Salicylate,
Ethylhexyl Methoxycinnamate
Butyl Methoxydibenzoylmethane
Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
2,4,6-tris-(4'-amino benzalmalonate de diisobutyle)-s-triazine
Anisotriazine,
Ethylhexyl triazone,
Diethylhexyl Butamido Triazone,
Methylène bis-Benzotriazolyl Tetramethylbutylphénol
Drometrizole Trisiloxane
Polysilicone-15
1,1-dicarboxy (2,2'-diméthyl-propyl)-4,4-diphénylbutadiène
2,4-bis-[5-1(diméthylpropyl)benzoxazol-2-yl-(4-phenyl)-imino]-6-(2-ethylhexyl)-imino-1,3,5-triazine
et leurs mélanges.

11. Composition selon la revendication 10, **caractérisée par le fait que** les filtres complémentaires inorganiques sont des pigments ou des nanopigments d'oxydes métalliques, enrobés ou non.

12. Composition selon la revendication 11, **caractérisée par le fait que** lesdits pigments ou nanopigments sont choisis parmi les oxydes de titane, de zinc, de fer, de zirconium, de cérium et leurs mélanges, enrobés ou non.

13. Composition selon l'une quelconque des revendications 1 à 12, **caractérisée par le fait qu'**elle comprend en outre au moins un agent de bronzage et/ou de brunissage artificiel de la peau.

14. Composition selon l'une quelconque des revendications 1 à 13, **caractérisée par le fait qu'**elle comprend en outre au moins un adjuvant choisi parmi les corps gras, les solvants organiques, les épaississants ioniques ou non ioniques, les adoucissants, les humectants, les antioxydants, les hydratants, les desquamants, les agents anti-radicalaires, les agents antipollution, les antibactériens, les agents anti-inflammatoires, les dépigmentants, les pro-pigmentants, les opacifiants, les stabilisants, les émollients, les silicones, les agents anti-mousse, les agents répulsifs contre les insectes, les parfums, les conservateurs, les tensioactifs anioniques, cationiques, non-ioniques, zwitterioniques ou amphotères, les antagonistes de substance P, les antagonistes de substance CGRP, les charges, les pigments, les polymères, les propulseurs, les agents alcalinisants ou acidifiants

15. Composition selon l'une quelconque des revendications 1 à 14, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une huile, d'une poudre, d'un bâtonnet solide, d'une mousse ou d'un spray.

16. Composition selon l'une quelconque des revendications 1 à 14, **caractérisée par le fait qu'**il s'agit d'une composition de maquillage des cils, des sourcils, des ongles ou de la peau et qu'elle se présente sous forme solide ou pâteuse, anhydre ou aqueuse, d'une émulsion, d'une suspension ou d'une dispersion.

17. Composition selon l'une quelconque des revendications 1 à 14, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

18. Utilisation d'un composé de formule (I) tel que défini dans l'une quelconque des revendications 1 à 4 dans une composition cosmétique ou dermatologique comme agent filtrant les radiations UV.

19. Utilisation d'un composé de formule (I) tel que défini dans l'une quelconque des revendications 1 à 4 dans une composition cosmétique comme agent de contrôle de la variation de la couleur de la peau due aux rayonnements UV.

20. Utilisation d'un composé de formule (I) tel que défini dans l'une quelconque des revendications 1 à 4 comme agent photostabilisant de polymères synthétiques ou de verres.

## Claims

1. Compound corresponding to the general formula (I) below: 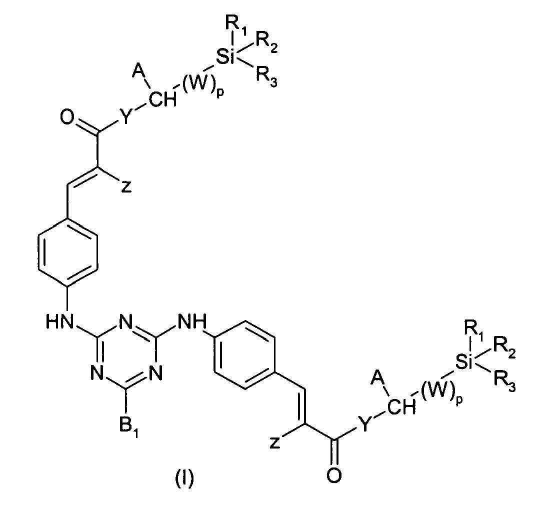 in which:
- the radicals R₁, R₂ and R₃, which may be identical or different, represent a linear or branched, saturated or unsaturated C₁-C₁₀ alkyl radical, containing one or more halogen atoms; a phenyl radical,
- p is equal to 0 or 1,
- A denotes hydrogen; a linear or branched, saturated or unsaturated C₁-C₈ alkyl radical; a phenyl radical; the group Si(CH₃)₃, with the proviso that when A is Si(CH₃)₃, then p = 0 and R₁, R₂ and R₃ are methyl,
- W is a linear or branched, saturated or unsaturated C₁-C₈ alkylene radical optionally substituted, with a hydroxyl radical,
- Z denotes -(C=O)OR₄, -(C=O)R₅, -(C=O)NR₆R₇, -SO₂R₈, -CN or -(C=O)YCHA(W)ₚSiR₁R₂R₃,
- the radical R₄ denotes hydrogen; a linear or branched, saturated or unsaturated C₁-C₂₀ alkyl radical,
- the radical R₅ denotes a linear or branched, optionally cyclic C₁-C₂₀ alkyl radical; a C₆-C₁₂ aryl,
- the radicals R₆ and R₇, which may be identical or different, represent hydrogen; a linear or branched C₁-C₂₀ alkyl,
- Y denotes -O- or -NR₇-,
- the radical R₈ denotes a linear or branched C₁-C₂₀ alkyl radical; a C₆-C₁₂ aryl,
- B₁ denotes a chromophoric group chosen from those of the para-aminobenzalmalonate, aminobenzimidazole, benzimidazole, aminobenzoate, aminosalicylate, anthranilate, aminobenzylidenecamphor, aminobenzotriazole, aminobenzoxazole or para-aminophenylbenzoxazole type;
- B₁ may denote a linear or branched C₁-C₂₀ aminoalkyl group; a C₆-C₂₀ aryl group optionally substituted with alkyl, hydroxyl and alkoxy radicals.

2. Compound according to Claim 1, in which B₁ denotes a group of formula (III) below: 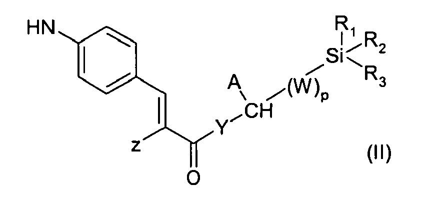 in which Z, A, p, W, R₁, R₂ and R₃ have the same meanings indicated in Claim 1.

3. Compound according to Claim 1 or 2, having at least one and even more preferentially all of the following characteristics:
- Z = -(C=O)OR₅, -CN or -(C=O)YCHA(W)ₚSiR₂R₃R₄,
- R₅ is methyl or ethyl,
- A is H,
- R₂ to R₄ denote C₁-C₄ alkyl and more preferentially methyl,
- n is 0,
- p is 0 or 1,
- W is a C₁-C₂ alkylene radical.

4. Compound according to Claim 3, chosen from the following compounds:

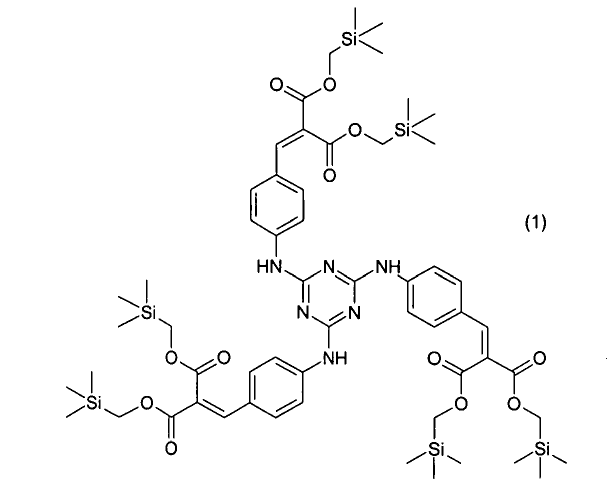

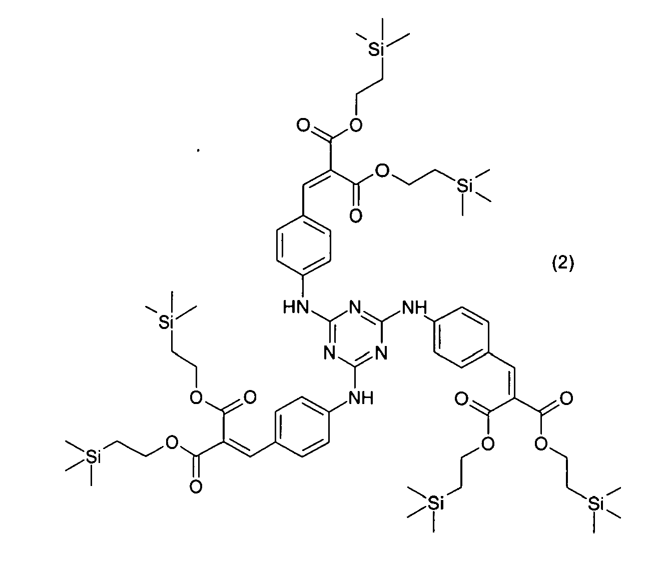

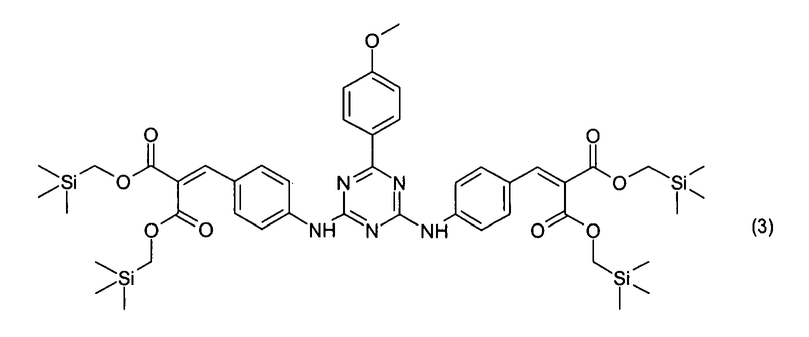

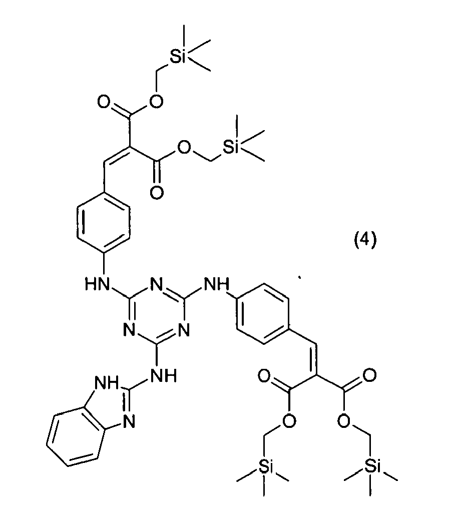

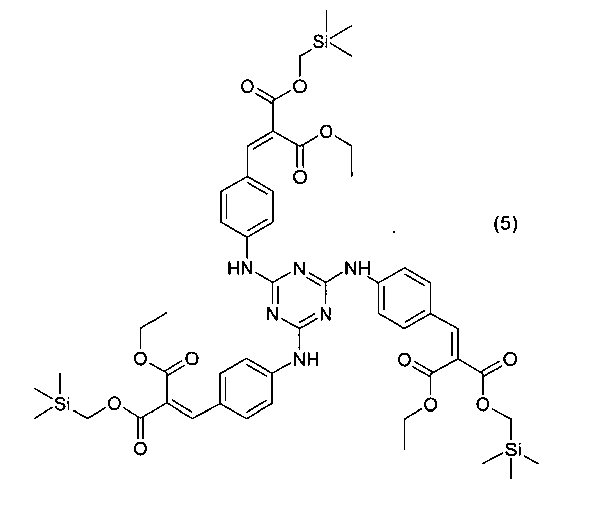

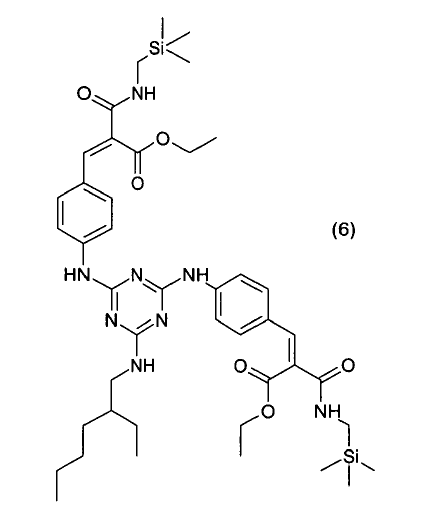

5. Cosmetic or dermatological composition for photoprotecting keratin materials, **characterized in that** it contains, in a cosmetically acceptable support, at least one compound of formula (I) as defined in any one of Claims 1 to 4.

6. Composition according to Claim 5, **characterized in that** the compound of formula (I) is present in the composition in a content ranging from 0.01% to 20% by weight relative to the total weight of the composition and preferably ranging from 0.1% to 10% by weight relative to the total weight of the composition.

7. Composition according to Claim 5 or 6,
**characterized in that** the said cosmetically acceptable support is in the form of an emulsion of oil-in-water or water-in-oil type.

8. Composition according to any one of Claims 5 to 7, **characterized in that** it also comprises one or more additional UV-A-active and/or UV-B-active organic or mineral screening agents.

9. Composition according to Claim 8, **characterized in that** the said additional organic screening agents are chosen from anthranilates; cinnamic derivatives; dibenzoylmethane derivatives; salicylic derivatives; camphor derivatives; triazine derivatives other than those defined in the preceding claims; benzophenone derivatives; β,β-diphenylacrylate derivatives; benzotriazole derivatives; benzalmalonate derivatives other than those defined in the preceding claims; benzimidazole derivatives; imidazolines; bis-benzazolyl derivatives; benzoxazole derivatives; p-aminobenzoic acid (PABA) derivatives; methylenebis(hydroxyphenyl-benzotriazole) derivatives; benzoxazole derivatives; screening polymers and screening silicones; dimers derived from α-alkylstyrene; 4,4-diarylbutadienes, and mixtures thereof.

10. Composition according to Claim 9, **characterized in that** the said additional organic screening agents are chosen from:
Ethylhexyl salicylate,
Ethylhexyl methoxycinnamate,
Butylmethoxydibenzoylmethane,
Octocrylene,
Phenylbenzimidazolesulfonic acid,
Benzophenone-3,
Benzophenone-4,
Benzophenone-5,
n-Hexyl 2-(4-diethylamino-2-hydroxybenzoyl)benzoate,
4-Methylbenzylidenecamphor,
Terephthalylidenedicamphorsulfonic acid,
Disodium phenyldibenzimidazoletetrasulfonate,
2,4,6-Tris(diisobutyl 4'-aminobenzalmalonate)-s-triazine,
Anisotriazine,
Ethylhexyltriazone,
Diethylhexylbutamidotriazone,
Methylenebis(benzotriazolyl)tetramethylbutylphenol,
Drometrizole trisiloxane,
Polysilicone-15
1,1-Dicarboxy(2,2'-dimethylpropyl)-4,4-diphenylbutadiene,
2,4-Bis[5-1(dimethylpropyl)benzoxazol-2-yl(4-phenyl)-imino]-6-(2-ethylhexyl)imino-1,3,5-triazine
and mixtures thereof.

11. Composition according to Claim 10, **characterized in that** the additional mineral screening agents are coated or uncoated metal oxide pigments or nanopigments.

12. Composition according to Claim 11, **characterized in that** the said pigments or nanopigments are chosen from titanium oxide, zinc oxide, iron oxide, zirconium oxide and cerium oxide, and mixtures thereof, which are coated or uncoated.

13. Composition according to any one of Claims 1 to 12, **characterized in that** it also comprises at least one agent for artificially tanning and/or browning the skin.

14. Composition according to any one of Claims 1 to 13, **characterized in that** it also comprises at least one adjuvant chosen from fatty substances, organic solvents, ionic or nonionic thickeners, softeners, humectants, antioxidants, moisturizers, desquamating agents, free-radical scavengers, antipollution agents, antibacterial agents, anti-inflammatory agents, depigmenting agents, propigmenting agents, opacifiers, stabilizers, emollients, silicones, antifoams, insect repellents, fragrances, preserving agents, anionic, cationic, nonionic, zwitterionic or amphoteric surfactants, substance P antagonists, CGRP antagonists, fillers, pigments, polymers, propellants and acidifying or basifying agents.

15. Composition according to any one of Claims 1 to 14, **characterized in that** it is a composition for protecting the human epidermis or an antisun composition and **in that** it is in the form of a nonionic vesicular dispersion, an emulsion, in particular an emulsion of oil-in-water type, a cream, a milk, a gel, a cream-gel, a suspension, a dispersion, an oil, a powder, a solid tube, a mousse or a spray.

16. Composition according to any one of Claims 1 to 14, **characterized in that** it is a composition for making up the eyelashes, the eyebrows, the nails or the skin and **in that** it is in solid or pasty, anhydrous or aqueous form or in the form of an emulsion, a suspension or a dispersion.

17. Composition according to any one of Claims 1 to 14, **characterized in that** it is a composition for protecting the hair against ultraviolet rays and **in that** it is in the form of a shampoo, a lotion, a gel, an emulsion or a nonionic vesicular dispersion.

18. Use of a compound of formula (I) as defined in any one of Claims 1 to 4, in a cosmetic or dermatological composition as an agent for screening out UV radiation.

19. Use of a compound of formula (I) as defined in any one of Claims 1 to 4, in a cosmetic composition as an agent for controlling the variation in skin colour caused by UV radiation.

20. Use of a compound of formula (I) as defined in any one of Claims 1 to 4, as an agent for photostabilizing synthetic polymers or glasses.

## Patentansprüche

1. Verbindung, die der folgenden allgemeinen Formel (I) entspricht: 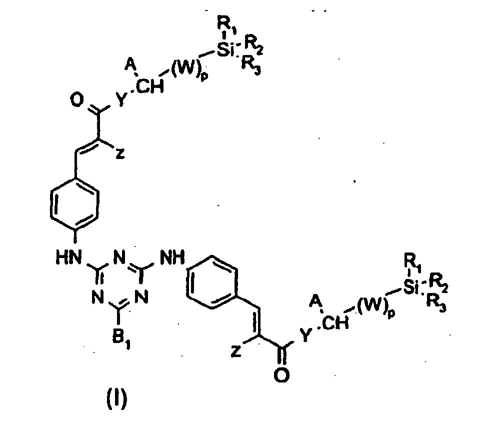 worin bedeuten:
- die Gruppen R₁, R₂ und R₃, die gleich oder verschieden sind, eine geradkettige oder verzweigte, gesättigte oder ungesättigte C₁₋₁₀-Alkylgruppe, die ein oder mehrere Halogenatome aufweisen kann; eine Phenylgruppe,
- p 0 oder 1,
- A Wasserstoff; eine geradkettige oder verzweigte, gesättigte oder ungesättigte C₁₋₈-Alkylgruppe; eine Phenylgruppe; die Gruppe Si(CH₃)₃, mit der Maßgabe, dass p = 0 und R₁, R₂ und R₃ Methyl bedeuten, wenn A Si(CH₃)₃ ist,
- W eine geradkettige oder verzweigte, gesättigte oder ungesättigte C₁₋₈-Alkylengruppe, die gegebenenfalls mit einer Hydroxygruppe substituiert ist,
- Z -(C=O)OR₄, -(C=O)R₅, -(C=O)NR₆R₇, -SO₂R₈, -CN oder
- (C=O)YCHA(W)ₚSiR₁R₂R₃,
- die Gruppe R₄ Wasserstoff; eine geradkettige oder verzweigte, gesättigte oder ungesättigte C₁₋₂₀-Alkylgruppe,
- die Gruppe R₅ eine geradkettige oder verzweigte, gegebenenfalls cyclische C₁₋₂₀-Alkylgruppe; eine C₆₋₁₂-Arylgruppe,
- die Gruppen R₆ und R₇, die gleich oder verschieden sind, Wasserstoff; eine geradkettige oder verzweigte C₁₋₂₀-Alkylgruppe,
- Y -O- oder -NR₇-,
- die Gruppe R₈ eine geradkettige oder verzweigte C₁₋₂₀-Alkylgruppe; eine C₆₋₁₂-Arylgruppe,
- B₁ eine chromophore Gruppe, die unter den Gruppen vom Typ *p*-Aminobenzalmalonat, Aminobenzimidazol, Benzimidazol, Aminobenzoat, Aminosalicylat, Anthranilat, Aminobenzylidencampher, Aminobenzotriazol, Aminobenzoxazol, *p*-Aminophenylbenzoxazol ausgewählt ist,
- B₁ kann eine geradkettige oder verzweigte C₁₋₂₀-Aminoalkylgruppe, eine ggf. mit Alkylgruppen, Hydroxygruppen und Alkoxygruppen substituierte C₆₋₂₀-Arylgruppe sein.

2. Verbindung nach Anspruch 1, worin B₁ eine Gruppe der folgenden Formel bedeutet: 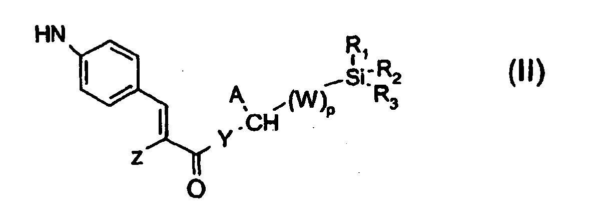 worin Z, A, p, W, R₁, R₂ und R₃ die in Anspruch 1 angegebenen Bedeutungen aufweisen.

3. Verbindung nach Anspruch 1 oder 2, die mindestens eine und vorzugsweise alle folgenden Eigenschaften besitzt:
- Z = -C=O)OR₅, -CN oder (C=O)YCHA(W)ₚSiR₂R₃R₄,
- R₅ bedeutet Methyl oder Ethyl,
- A ist H,
- R₂ bis R₄ bedeuten C₁₋₄-Alkyl und vorzugsweise Methyl,
- n ist 0,
- p ist 0 oder 1,
- W ist eine C₁₋₂-Alkylengruppe.

4. Verbindung nach Anspruch 3, die unter den folgenden Verbindungen ausgewählt ist:

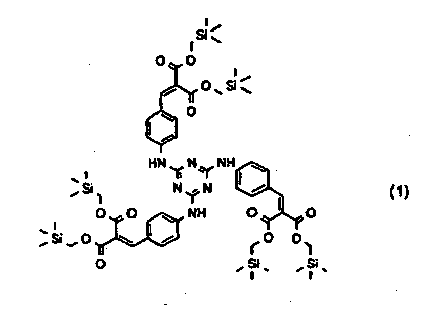

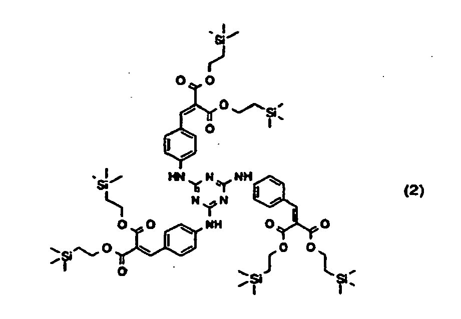

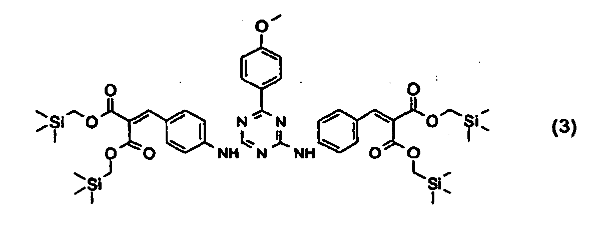

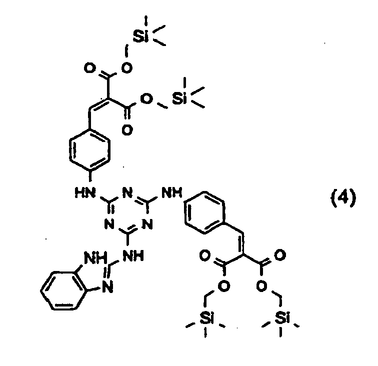

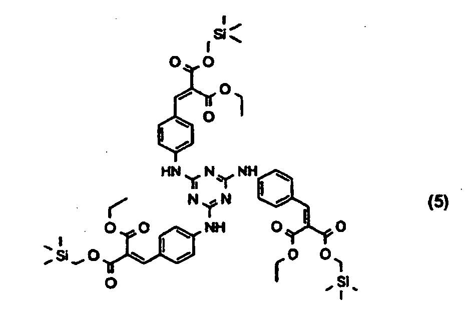

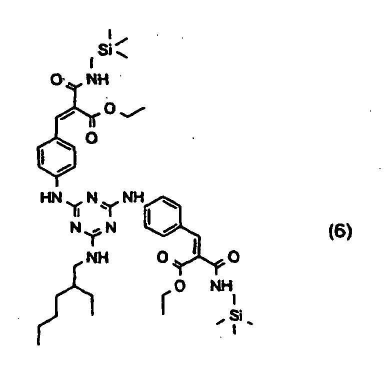

5. Kosmetische oder dermatologische Zusammensetzung, die für den Lichtschutz von Keratinsubstanzen vorgesehen ist, **dadurch gekennzeichnet, dass** sie in einem kosmetisch akzeptablen Träger mindestens eine Verbindung der Formel (I) enthält, wie sie in einem der Ansprüche 1 bis 4 definiert ist.

6. Zusammensetzung nach Anspruch 5, **dadurch gekennzeichnet, dass** die Verbindung der Formel (I) in der Zusammensetzung in einem Mengenanteil von 0,01 bis 20 Gew.-%, bezogen auf das Gesamtgewicht der Zusammensetzung, und vorzugsweise 0,1 bis 10 Gew.-%, bezogen auf das Gesamtgewicht der Zusammensetzung, enthalten ist.

7. Zusammensetzung nach Anspruch 5 oder 6, **dadurch gekennzeichnet, dass** der kosmetisch akzeptable Träger als Emulsion vom Typ einer Öl-in-Wasser-Emulsion oder einer Wasser-in-Öl-Emulsion vorliegt.

8. Zusammensetzung nach einem der Ansprüche 5 bis 7, **dadurch gekennzeichnet, dass** sie ferner ein oder mehrere zusätzliche, im UV-A- und/oder UV-B-Bereich wirksame, organische oder anorganische Filter enthält.

9. Zusammensetzung nach Anspruch 8, **dadurch gekennzeichnet, dass** die zusätzlichen organischen Filter unter Anthranilaten; Zimtsäurederivaten; Dibenzoylmethanderivaten; Salicylsäurederivaten; Campherderivaten; Triazinderivaten, die von den in den vorhergehenden Ansprüchen definierten Verbindungen verschieden sind; Benzophenonderivaten; β, β-Diphenylacrylatderivaten; Benzotriazolderivaten; Benzalmalonatderivaten, die von den in den vorhergehenden Ansprüchen definierten Verbindungen verschieden sind; Benzimidazolderivaten; Imidazolinderivaten; Bis-benzoazolylderivaten; Benzoxazolderivaten; Derivaten von p-Aminobenzoesäure (PABA); Methylen-bis(hydroxy-phenylbenzotriazol)derivaten; Benzoxazolderivaten; polymeren Filtern und Siliconfiltern; von α-Alkylstyrol abgeleiteten Dimeren; 4,4-Diarylbutadienen und deren Gemischen ausgewählt sind.

10. Zusammensetzung nach Anspruch 9, **dadurch gekennzeichnet, dass** die zusätzlichen organischen Filter ausgewählt sind unter:
Ethylhexyl Salicylate,
Ethylhexyl Methoxycinnamate,
Butyl Methoxydibenzoylmethane,
Octocrylene,
Phenylbenzimidazole Sulfonic Acid,
Benzophenone-3,
Benzophenone-4,
Benzophenone-5,
*n*-Hexyl-2-(4-diethylamino-2-hydroxybenzoyl)-benzoat,
4-Methylbenzylidene Camphor,
Terephthalylidene Dicamphor Sulfonic Acid,
Disodium Phenyl Dibenzimidazole Tetra-sulfonate,
2,4,6-Tris-(diisobutyl-4'-aminobenzalmalonat)-s-triazin,
Anisotriazine,
Ethylhexyl Triazone,
Diethylhexyl Butamido Triazone,
Methylene bis-Benzotriazolyl Tetramethylbutylphenol,
Drometrizole Trisiloxane,
Polysilicone-15,
1, 1-Dicarboxy-(2,2'-dimethylpropyl)-4,4-diphenylbutadien,
2,4-Bis[5-1 (dimethylpropyl)benzoxazol-2-yl-(4-phenyl)-imino]-6-(2-ethylhexyl)imino-1,3,5-triazin,
und deren Gemischen.

11. Zusammensetzung nach Anspruch 10, **dadurch gekennzeichnet, dass** die zusätzlichen anorganischen Filter Pigmente oder Nanopigmente von Metalloxiden sind, die gegebenenfalls umhüllt sind.

12. Zusammensetzung nach Anspruch 11, **dadurch gekennzeichnet, dass** die Pigmente oder Nanopigmente unter den Oxiden von Titan, Zink, Eisen, Zirconium, Cer und deren Gemischen ausgewählt sind, wobei sie umhüllt oder nichtumhüllt sind.

13. Zusammensetzung nach einem der Ansprüche 1 bis 12, **dadurch gekennzeichnet, dass** sie ferner mindestens ein Bräunungsmittel und/oder Mittel zur künstlichen Braunfärbung der Haut enthält.

14. Zusammensetzung nach einem der Ansprüche 1 bis 13, **dadurch gekennzeichnet, dass** sie ferner mindestens einen Zusatzstoff enthält, der unter den Fettsubstanzen, organischen Lösungsmitteln, ionischen oder nichtionischen Verdickungsmitteln, beruhigenden Stoffen, Feuchthaltemitteln, Antioxidantien, Hydratisierungsmitteln, abschuppenden Wirkstoffen, Radikalfängern für freie Radikale, Antipollutionswirkstoffen, antibakteriellen Wirkstoffen, entzündungshemmenden Wirkstoffen, Depigmentierungsmitteln, pigmentierungsfördernden Wirkstoffen, Trübungsmitteln, Stabilisatoren, Emollientien, Siliconen, Schaumverhütungsmitteln, Insekten abwehrenden Wirkstoffen, Parfums, Konservierungsmitteln, anionischen, kationischen, nichtionischen, zwitterionischen oder amphoteren grenzflächenaktiven Stoffen, Substanz P-Antagonisten, Substanz CGRP-Antagonisten, Füllstoffen, Pigmenten, Polymeren, Treibmitteln, Alkalisierungsmitteln oder Ansäuerungsmitteln ausgewählt ist.

15. Zusammensetzung nach einem der Ansprüche 1 bis 14, **dadurch gekennzeichnet, dass** es sich um eine Zusammensetzung zum Schutz der menschlichen Epidermis oder ein Sonnenschutzmittel handelt und sie in Form einer nichtionischen Vesikeldispersion, als Emulsion und insbesondere Emulsion vom Öl-in-Wasser-Typ, Creme, Milch, Gel, Gelcreme, Suspension, Dispersion, Öl, Pulver, fester Stift, Schaum oder Spray vorliegt.

16. Zusammensetzung nach einem der Ansprüche 1 bis 14, **dadurch gekennzeichnet, dass** es sich um eine Zusammensetzung zum Schminken der Wimpern, der Augenbrauen, der Nägel oder der Haut handelt und sie in fester oder pastöser, wässriger oder wasserfreier Form, als Emulsion, Suspension oder Dispersion vorliegt.

17. Zusammensetzung nach einem der Ansprüche 1 bis 14, **dadurch gekennzeichnet, dass** es sich um eine Zusammensetzung handelt, die zum Schutz der Haare gegen UV-Strahlung vorgesehen ist, und dass sie in Form eines Haarwaschmittels, einer Lotion, eines Gels, einer Emulsion oder einer nichtionischen Vesikeldispersion vorliegt.

18. Verwendung einer Verbindung der Formel (I) nach einem der Ansprüche 1 bis 4 in einer kosmetischen oder dermatologischen Zusammensetzung als UV-Filter.

19. Verwendung einer Verbindung der Formel (I) nach einem der Ansprüche 1 bis 4 in einer kosmetischen Zusammensetzung, als Stoff, um die Veränderung der Farbe der Haut, die durch die UV-Strahlung verursacht wird, zu kontrollieren.

20. Verwendung einer Verbindung der Formel (I) nach einem der Ansprüche 1 bis 4 als Mittel zur Fotostabilisierung von synthetischen Polymeren oder Gläsern.
